# FASCICULE DE BREVET EUROPEEN

(11) **EP 0 520 888 B1**
(45) Date de publication et mention de la délivrance du brevet: **19.09.2001**
(21) Numéro de dépôt: 92401788.2
(22) Date de dépôt: 25.06.1992
(51) Int. Cl.: A61K 31/765, A61K 9/51, A61K 47/34

(54) **Nanoparticules à base d'un copolymère à blocs de polyoxyde d'éthylène et acide polylactique**
Nanopartikel aus Polyoxyäthylen-Polymilchsäure Blockcopolymeren
Polyoxyethylene-polylactic acid block copolymer nanoparticles

(30) Priorité: 28.06.1991 FR 9108041
(43) Date de publication de la demande: 30.12.1992
(73) Titulaire: Aventis Pharma S.A., 92160 Antony (FR)
(72) Inventeur: Spenlehauer, Gilles, F-94230 Cachan (FR); Bazile, Didier, F-94210 La Varenne St. Hilaire (FR); Veillard, Michel, F-92330 Sceaux (FR); Prud'homme, Christian, F-69006 Lyon (FR); Michalon, Jean-Paul, F-69008 Lyon (FR)
(74) Mandataire: Le Pennec, Magali

(56) Documents cités:
- WO-A-78/00011
- STN INTERNATIONAL INFORMATION SERVICES BASE DE DONNEES: CHEMICAL ABSTRACTS, vol. 113, no. 18, abrégé no. 153222a, Columbus, Ohio, US; P. ARTURSSON et al.: "Preparation of sterically stabilized nanoparticles by desolvation from graft copolymers", & J. POLYM. SCI., PART A: POLYM. CHEM., 1990, 28(10), 2651-63

## Description

La présente invention concerne de nouvelles particules sphériques de petites dimensions, souvent inférieures à 500 nm. Les nouvelles particules de la présente invention, encore appelées nanoparticules présentent l'avantage de pouvoir circuler dans le flux sanguin sans problème de dimension au niveau des capillaires et présentent en plus l'avantage de pouvoir éviter le système réticulo endothélial. L'invention vise aussi l'utilisation des nouvelles particules selon l'invention en pharmacie humaine ou animale.

Les nanoparticules qui peuvent être utilisées pour une injection dans le système vivant doivent être biocompatibles. Ainsi tous les systèmes polymériques ne contenant pas de chaînes polymériques biodégradables ou biorésorbables ne sont pas acceptables pour de telles injections. Il est d'autre part préférable lorsque l'on utilise de tels systèmes que les produits de dégradation soient compatibles avec les organismes vivants. A ce jour seulement deux types de polymères peuvent convenir; il s'agit des polymères lactiques ou glycoliques ou des copolymères mixtes lactique-glycolique.

Les procédés de préparation des nanoparticules peuvent être divisés en trois types de procédé, le premier consistant à polymériser les monomères et à former les nanoparticules simultanément, les deux autres consistant à solubiliser le polymère et à former les nanoparticules indépendamment.

Le premier type de procédé consiste à effectuer une polymérisation d'un monomère dans une solution de façon à obtenir une dispersion micellaire du polymère dans la solution. Ce type de procédé est limité à des monomères polymérisables en solution, il nécessite l'élimination après l'étape de polymérisation du catalyseur de polymérisation, des oligomères de bas poids moléculaire, des monomères et des agents surfactants nécessaires à la polymérisation. Le polymère obtenu présente une répartition de poids moléculaire aléatoire.

Le deuxième et le troisième types de procédé consistent à utiliser des polymères préformés, à les solubiliser dans un solvant, à former un précipité ou une dispersion à partir d'une solution de ces polymères et d'un non solvant puis à évaporer le solvant de façon à récupérer les nanoparticules sous forme d'une suspension colloïdale. La solution solvante est généralement une solution organique du polymère, la solution non solvante est souvent une solution aqueuse.

Selon le deuxième type de procédé, le polymère est solubilisé dans un solvant organique miscible à l'eau. Lorsque cette solution est mélangée à la phase aqueuse, le polymère insoluble dans le mélange phase aqueuse/solvant organique précipite sous forme de nanoparticules.

Selon le troisième type de procédé on émulsionne le solvant organique contenant le polymère, non miscible à l'eau, dans une phase aqueuse puis on évapore le solvant organique.

La formation du précipité ou de l'émulsion exige la présence d'une quantité d'agent surfactant loin d'être négligeable. Or lors de l'évaporation subséquente, il est très difficile d'éliminer l'agent surfactant qui subsiste dans la suspension colloïdale de nanoparticules obtenue, cet agent est souvent indésirable pour une bonne biocompatibilé. Ces deux dernières techniques ne sont donc pas utilisables pour la préparation de nanoparticules biocompatibles à cause de la présence de l'agent protecteur colloïdal.

Dans le brevet EP 166596 ont été décrits des copolymères autodispersables dans l'eau pour former une dispersion stable. Ces copolymères peuvent comprendre des motifs polylactiques et des motifs polyéthylèneglycol.

La présente invention concerne de nouvelles nanoparticules évitant le système réticulo endothélial à base de polymères comportant une majorité de motifs dégradables et ne contenant éventuellement pas d'agent tensioactif additionnel. Elle sont obtenues à partir d'un copolymère constitué d'une majorité de motifs polylactiques et d'une minorité de motifs oxyde d'éthylène et/ou oxyde de propylène. Ce copolymère répond pour la majorité de ses motifs de préférence à la formule (I) suivante : 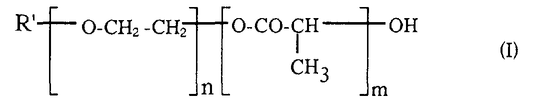 dans laquelle :
R' représente l'hydrogène ou un groupe alkyle contenant 1 à 4 atomes de carbone,
n est un nombre entier égal à 48
m est un nombre entier compris entre 40 et 150,
étant entendu que ledit copolymère n'est pas choisi parmi les copolymères auto-dispersables dans l'eau pour former une dispersion stable.

Le motif polymérique polylactique de ce copolymère de formule (I) a de préférence un poids moléculaire compris entre 700 et 100 000, le motif polyoxyde d'éthylène a lui de préférence un poids moléculaire compris entre 1 000 et 40 000. D'une façon encore plus préférentielle le motif polymérique polylactique a un poids moléculaire compris entre 1 000 et 60 000, le motif polyoxyde d'éthylène a un poids moléculaire compris entre 1 000 et 6 000.

Selon une dernière préférence le polymère polylactique est un polymère contenant 50 % de motifs lactique de configuration D (PLA₅₀) et le polyoxyde d'alkylène est un polyoxyde d'éthylène.

Ce copolymère se présente sous une forme dibloc, c'est à dire que selon une manière pratique de mise en oeuvre on part d'un polyéthylène et/ou polyoxyde de propylène commercial monofonctionnel de poids moléculaire désiré c'est à dire d'un poids moléculaire compris entre 1000 et 40 000 ou encore comportant 20 à 1000 motifs oxyde d'éthylène ou de propylène, de préférence comportant 20 à 150 motifs oxyde d'éthylène ou 20 à 100 motifs oxyde de propylène sur lequel on greffe des motifs lactides jusqu'à obtenir le poids moléculaire désiré sur la chaîne polylactique en présence d'un initiateur tel que notamment l'octoate d'étain.

On peut préciser que pour obtenir des blocs polylactiques d'un poids moléculaire compris entre 1 000 et 60 000, il est souhaitable d'introduire entre environ 10 et 1000 motifs lactide. On préfère tout particulièrement utiliser des copolymères polyoxyde d'éthylène et/ou polyoxyde de propylène polylactique dont la chaîne contient entre 10 et 150 motifs lactiques.

On préfère encore plus particulièrement partir d'un polyéthylène glycol commercial comportant 48 motifs oxyde d'éthylène d'un poids moléculaire de 2100 que l'on fait réagir avec 40 à 150 motifs lactide.

Ces nouvelles nanoparticules évitant le système réticulo endothélial peuvent aussi être constituées d'un mélange d'un ou de plusieurs polymère(s) polylactique pur et du copolymère de formule (I).

Afin de préparer ces dernières nouvelles nanoparticules selon l'invention il est nécessaire de mélanger le copolymère de formule (I) avec une quantité utile d'un polymère polylactique. Ce polymère polylactique est de préférence un polymère comportant un mélange 50/50 d'isomère D et L de l'acide lactique (PLA₅₀). On préfère utiliser un mélange contenant entre 10 et 80 % en poids de copolymère de formule (I) par rapport au polymère polylactique. Le rapport pondéral final dans la composition polymérique entre le motif polyoxyde d'éthylène et/ou de propylène et les motifs polylactique est de préférence compris entre 1 et 25 % en poids. On préfère tout particulièrement utiliser la composition obtenue par mélange d'un polymère polylactique de poids moléculaire de 60 000, d'un copolymère de formule (I) dans lequel R représente l'hydrogène, n est égal à 48 et m est égal à 133.

Selon une première méthode de préparation des nanoparticules, le copolymère polylactique-polyoxyde d'éthylène et/ou de propylène désiré, éventuellement en mélange avec le polymère polylactique est mis en solution dans un solvant ou dans un mélange de solvants, puis la solution organique est versée dans une solution aqueuse, de façon à provoquer la formation des nanoparticules par précipitation. Dans ce procédé, on n'utilise éventuellement pas d'agent protecteur colloïdal additionnel. On entend par agent protecteur colloïdal, les agents de surface dont font partie les agents tensioactifs qui favorisent la formation de colloïdes.

Le solvant ou le mélange de solvants solubilisant le copolymère est choisi parmi les cétones telle que l'acétone, les éthers cycliques tels que le tétrahydrofurane, les dioxanes; les nitriles tels que l'acétonitrile. On préfère utiliser l'acétone. La solubilité du copolymère dans ces solvants est de préférence supérieure à 10 g/l.

La solution aqueuse peut être de l'eau pure ou une solution saline telle par exemple une solution tampon ou également.une solution de glucose.

Le rapport volumique entre la solution aqueuse et la solution du copolymère est compris de préférence entre 0,5 et 10, et tout particulièrement entre 1 et 10. La quantité de copolymère introduite dans le solvant dépend bien entendu de sa solubilité, mais pour une meilleure mise en oeuvre de l'invention, c'est à dire essentiellement pour obtenir un rendement optimum en nanoparticules formées, une quantité comprise entre 10 et 50 mg/ml est préférée.

Selon une deuxième méthode de préparation des nanoparticules, le polymère polylactique polyoxyde d'éthylène et/ou de propylène est mis en solution dans un ester, de préférence dans l'acétate d'éthyle puis la solution organique est versée dans la solution aqueuse. Les nonoparticules sont formées par l'utilisation d'un microfluidiseur.

Le solvant du copolymère est ensuite évaporé en chauffant la solution colloïdale de nanoparticules au dessus de la température d'ébullition du solvant dans le cas où l'élimination est effectuée à pression atmosphérique ou à une température inférieure si l'évaporation est effectuée sous pression réduite. Après que le solvant ait été éliminé, la suspension de nanoparticules dans l'eau est filtrée sur un filtre de diamètre de pores d'environ 1 µm, de façon à éliminer les agrégats et les grosses particules. Le rendement en nanoparticules obtenues dépasse généralement 50 %.

La formation de nanoparticules peut être effectuée en présence d'un principe actif pharmaceutique qui peut être introduit soit dans le solvant du copolymère soit dans le solvant de précipitation, il doit de préférence être soluble dans le solvant du polymère et non soluble dans l'eau, bien qu'il soit toujours possible de former des nanoparticules si le principe actif est soluble dans l'eau, le rendement en sera néanmoins amoindri.

Les nanoparticules obtenues ne contiennent que le copolymère de formule (I) ou le mélange de polymères polylactiques et de copolymère de formule (I) et éventuellement un principe actif si la précipitation est effectuée en présence d'un principe actif. Elles présentent un diamètre moyen compris entre 50 et 500 nm et de préférence un diamètre moyen compris entre 50 et 250 nm.

Les nanoparticules obtenues sont utilisées pour des injections dans un organisme vivant dans la mesure où leur avantage essentiel est de pouvoir éviter le système réticulo endothélial, ainsi leur application principale se trouve en pharmacie humaine ou animale.ou pour le diagnostic médical. Ces produits sont injectables par voie intramusculaire, sous cutanée, intraartérielle, intraveineuse, intraorgane ou intracavités sans risque anaphylactique

L'invention sera plus complètement décrite à l'aide des exemples suivants qui ne doivent pas être considérés comme limitatifs de l'invention.

### EXEMPLE 1

### Préparation de copolymères polylactique polyéthylène glycol

### 1.1) polymère PLA²⁹⁰⁰- PEG²¹⁰⁰

Dans un ballon tricol de 250 ml muni d'un agitateur à palettes et d'un réfrigérant ascendant avec circulation d'azote sec, le ballon étant chauffé par un bain d'huile régulé, on introduit:

| | |
|---|---|
| dl lactide | 144 g |
| polyéthylène glycol | 79.3 g |
| octoate stanneux | 0.256 g |
| toluène distillé | 335 g |

Le lactide est recristallisé la veille dans l'acétate d'éthyle, puis lavé le jour même à l'éther éthylique. On le séche sous vide. On charge tous les réactifs puis on chauffe sous léger reflux (110-114°C) pendant 5 heures et demi. Le solvant est ensuite éliminé sous vide à l'aide d'un évaporateur rotatif (40 mm de Hg - 100°C).

On obtient 226.3 g de concentrat.

La purification du copolymère est effectuée de la façon suivante:

On charge :

| | |
|---|---|
| concentrat | 215 g |
| dichlorométhane | 280 g |

On agite jusqu'à formation d'une solution homogène. Cette solution est coulée lentement dans 900 ml d'hexane à froid. Le polymère précipite sous forme d'une pâte qui est séparée par décantation. Le catalyseur de polymérisation est éliminé dans la phase hexanique. Aprés séparation du polymère il est mis à sécher dans une étuve sous vide à 40 °C. On obtient 188.4 g de copolymère dont la masse est analysée par résonnance magnétique nucléaire, la masse de polyéthylène glycol est de 2100 et celle de polylactique de 2900, ce qui représente 40 motifs lactique et 48 motifs oxyde d'éthylène

### 1.2) polymère PLA⁹⁶⁰⁰-PEG²¹⁰⁰

On reproduit l'exemple 1.1 en introduisant les composés suivants :

| | |
|---|---|
| dl lactide | 48.6 g |
| polyéthylène glycol | 10 g |
| octoate stanneux | 0.085 g |
| toluène distillé | 90 g |

après réaction on obtient 63,6 g de concentrat que l'on purifie par la méthode suivante : on met en solution 40 g de concentrat dans 200 g de dichlorométhane jusqu'à l'obtention d'une solution homogène. Cette solution est versée lentement dans 800 ml d'eau maintenu entre 55 et 60°C. Le polymère précipite et le dichlorométhane est évaporé, le lactide non réagi reste en solution aqueuse, le polymère est centrifugé puis séché à l'étuve sous vide à 40°C.

On obtient 35g de polymère dont l'analyse par résonnance magnétique nucléaire permet de déterminer le poids moléculaire. Ce dernier est de 9600 pour la chaîne lactique et de 2100 pour la chaîne polyoxyde d'éthylène, ce qui représente 133 motifs lactique et 48 motifs oxyde d'éthylène.

### 1.3) polymère PLA⁴⁰⁰⁰⁰

Dans un réacteur d'un litre chauffé par un bain d'huile et muni d'un agitateur sous forme d'une ancre et d'un réfrigérant ascendant et maintenu sous azote, on introduit 180 g de xylène distillé avant emploi et 0,180 g d'octoate d'étain, on chauffe, puis on introduit 120 g de dl lactide S de la société Boehringer préalablement recristallisé dans l'acétate d'éthyle et lavé à l'éther sulfurique.

On laisse réagir 5 heures à 140°C, en fin de réaction, on refroidit rapidement puis on élimine une partie du xylène sous vide. On dissout le polymère dans le dichlorométhane et on le précipite par le méthanol. On le sèche dans une étuve à vide à 65°C.

### EXEMPLE 2

### Préparation de nanoparticules à partir de ces polymères par la première méthode de préparation

On utilise 50 mg d'un mélange selon le tableau suivant de copolymère préparé en 1.1, de polymère polylactique selon 1.3 et d'un polymère polylactique de poids moléculaire 18 000 marqué au carbone 14 que l'on dissout dans 5 ml d'acétone. Un essai comparatif est effectuée en utilisant des nanoparticules préparées selon l'art antérieur à partir du même polymère polylactique mais en présence d'un agent protecteur colloïdal qui est le Pluronic F68 ou Poloxamer 188. Les nanoparticules sont préparées par précipitation en versant lentement ce volume dans 5 ml de tampon phosphate 0,13 molaire (pH 7,4). La suspension colloïdale obtenue est évaporée 30 minutes au rotavapor à température ambiante et sous une pression de 3 mm de Hg. La suspension est alors filtrée sur un filtre de 1,2 µm afin d'éliminer les grosses particules et les aggrégats.

On injecte à chaque rat 400 µl de suspension, les rats sont répartis en lots de cinq, un pour chaque concentration en polyoxyde d'éthylène. La cinétique de capture des particules par le système réticulo endothélial est représentée en portant la radioactivité restant dans le plasma en % de la radioactivité présente dans le plasma en fin de perfusion en fonction du temps. La demi vie des particules en fonction de la quantité de polyéthylène glycol introduite est indiquée dans le tableau ci-dessous. Les courbes de radioactivité restante dans le plasma sont tracées en annexe.

| % PEG | 0 | 0 | 1.6 | 3.2 | 6.6 | 9.8 | 13.1 |
|---|---|---|---|---|---|---|---|
| PLA⁹⁶⁰⁰PEG²¹⁰⁰ | 0 | 0 | 9 | 18 | 37 | 55 | 73 |
| PLA^{40 000} | 70 | 0 | 61 | 55 | 36 | 18 | 0 |
| ¹⁴C PLA^{18 000} | 30 | 100 | 30 | 27 | 27 | 27 | 27 |
| tensioactif F68 50g/l | OUI | NON | NON | NON | NON | NON | NON |
| T1/2 vie en mn | 1.9 | 1.8 | 2.3 | 5.2 | 10.0 | 18.7 | 43 |

### Préparation des nanoparticules à partir de ces polymères par la deuxième méthode de préparation

Sans agent tensio-actif.

On utilise 100 mg de PLA₅₀⁹⁶⁰⁰PEG²¹⁰⁰ et 10 mg de poly(d,l-acide lactique) de poids moléculaire 18000 marqué au ¹⁴C que l'on dissout dans 1 ml d'acétate d'éthyle. Cette solution est ensuite dispersée à l'aide d'un ultraturrax dans 10 ml d'eau. Une émulsion grossière est obtenue. Elle est ensuite recyclée pendant 2 minutes à l'aide d'un homogénéisateur haute pression type MICROFLUIDICS. L'émulsion est débarrassée de l'acétate d'éthyle à l'aide d'un évaporateur rotatif à une pression de 50.5 cm de mercure à 20°C. Le pseudolatex obtenu est constitué de nanoparticules d'un diamètre moyen de 145 ± 60 nm. La demi-vie de ces nanoparticules dans le sang est de 1 heure.

Avec un agent tensio-actif.

On procède de même que dans l'exemple précédent en dissolvant 50 mg de PLA⁹⁶⁰⁰PEG²¹⁰⁰, 50 mg de PLA_{d,l}⁵²⁰⁰⁰ et 10 mg de PLA_{d,l}¹⁸⁰⁰⁰ marqué au ¹⁴C que l'on dissout dans 1 ml d'acétate d'éthyle. La phase aqueuse est une solution de cholate de sodium à 10 g.l⁻¹ dans l'eau. Diamètre des nanoparticules : 105 ± 45 nm. Demie-vie: 0.5 heure.

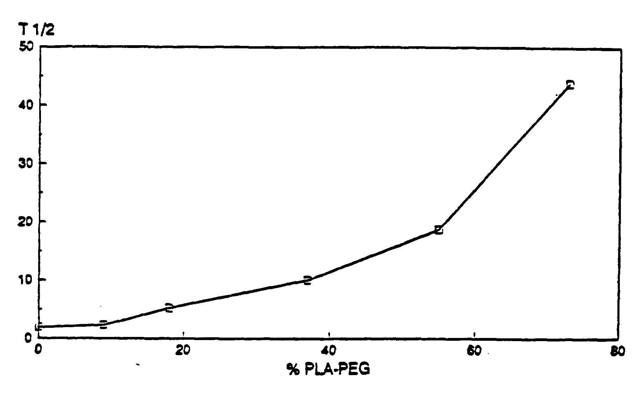

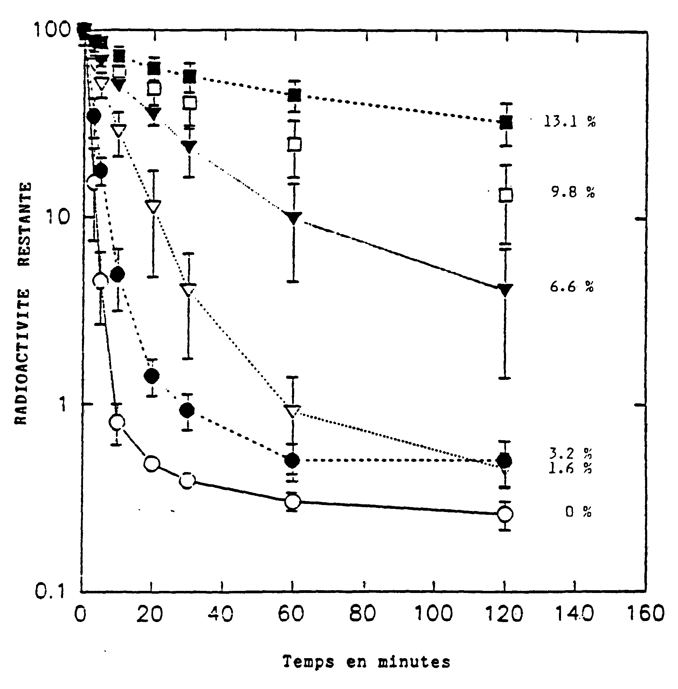

## Revendications

1. Nanoparticules évitant le système réticulo endothélial **caractérisées en ce qu'**elles sont constituées d'un copolymère dibloc polylactique / polyoxyde d'éthylène comportant une majorité de motifs de formule : 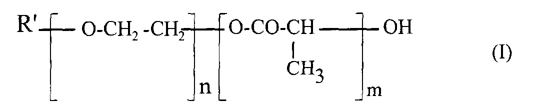 dans laquelle :
R' représente l'hydrogène ou un groupe alkyle contenant 1 à 4 atomes de carbone,
n est un nombre entier égal à 48
m est un nombre entier compris entre 40 et 150,
étant entendu que ledit copolymère n'est pas choisi parmi les copolymères auto-dispersables dans l'eau pour former une dispersion stable.

2. Nanoparticules évitant le système réticulo endothélial, **caractérisées en ce qu'**elles sont constituées d'un mélange d'un copolymère défini selon la revendication 1 et d'un ou plusieurs polymère(s) polylactique.

3. Nanoparticules selon la revendication 1 ou 2, **caractérisées en ce qu'**elles comprennent un principe actif pharmaceutique.

4. Nanoparticules selon l'une quelconque des revendications 1 à 3 **caractérisées en ce que** le copolymère est un copolymère polylactique / polyéthylène glycol : PLA²⁹⁰⁰ - PEG²¹⁰⁰.

5. Nanoparticules selon l'une quelconque des revendications 1 à 3 **caractérisées en ce que** le copolymère est un copolymère polylactique / polyéthylène glycol : PLA⁹⁶⁰⁰ - PEG²¹⁰⁰.

6. Nanoparticules selon l'une quelconque des revendications 1 à 5 **caractérisées en ce qu'**elles présentent une dimension moyenne comprise entre 50 et 500 nm

7. Nanoparticules selon la revendication 6 **caractérisées en ce qu'**elles présentent une dimension moyenne comprise entre 50 et 250 nm.

8. Application des nanoparticules selon l'une quelconque des revendications 1 à 7, pour la préparation d'un médicament destiné à la pharmacie humaine ou animale.

## Patentansprüche

1. Nanopartikel, die das retikulo-endotheliale System umgehen, **dadurch gekennzeichnet, dass** sie aus einem Polymilchsäure/Polyethylenoxid-Diblock-Copolymer bestehen, das eine Überzahl an Einheiten der Formel: 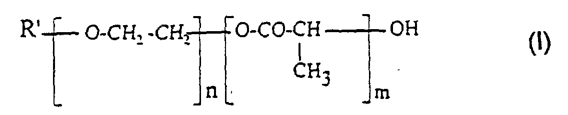 umfasst, in der:
R' ein Wasserstoffatom oder eine Alkylgruppe mit 1 bis 4 Kohlenstoffatomen darstellt,
n eine ganze Zahl gleich 48 ist,
m eine ganze Zahl zwischen 40 und 150 ist,
wobei es sich versteht, dass besagtes Copolymer nicht unter den Copolymeren, die zur Bildung einer stabilen Dispersion in Wasser autodispergierbar sind, ausgewählt ist.

2. Nanopartikel, die das retikulo-endotheliale System umgehen, **dadurch gekennzeichnet, dass** sie aus einem Gemisch eines gemäß Anspruch 1 definierten Copolymers und eines oder mehrerer Polymilchsäurepolymere bestehen.

3. Nanopartikel gemäß Anspruch 1 oder 2, **dadurch gekennzeichnet, dass** sie einen pharmazeutischen Wirkstoff umfassen.

4. Nanopartikel gemäß einem der Ansprüche 1 bis 3, **dadurch gekennzeichnet, dass** das Copolymer ein Polymilchsäure/Polyethylenglykol-Copolymer: PLA²⁹⁰⁰ - PEG²¹⁰⁰ ist.

5. Nanopartikel gemäß einem der Ansprüche 1 bis 3, **dadurch gekennzeichnet, dass** das Copolymer ein Polymilchsäure/Polyethylenglykol-Copolymer: PLA⁹⁶⁰⁰ - PEG²¹⁰⁰ ist.

6. Nanopartikel gemäß einem der Ansprüche 1 bis 5, **dadurch gekennzeichnet, dass** sie eine mittlere Größe zwischen 50 und 500 nm aufweisen.

7. Nanopartikel gemäß Anspruch 6, **dadurch gekennzeichnet, dass** sie eine mittlere Größe zwischen 50 und 250 nm aufweisen.

8. Anwendung der Nanopartikel gemäß einem der Ansprüche 1 bis 7 für die Herstellung eines Medikaments für die Human- oder Tiermedizin.

## Claims

1. Nanoparticles which evade the reticuloendothelial system, **characterized in that** they consist of a polylactic/polyethylene oxide diblock copolymer comprising a majority of units of formula: 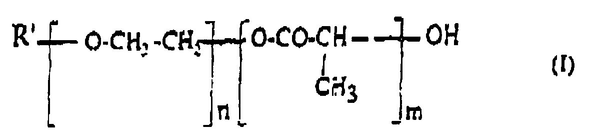 in which:
R' represents hydrogen or an alkyl group containing 1 to 4 carbon atoms,
n is an integer equal to 48,
m is an integer between 40 and 150,
it being understood that the said copolymer is not chosen from copolymers that are self-dispersible in water to form a stable dispersion.

2. Nanoparticlea which evade the reticulo-endothelial system, **characterized in that** they consist of a mixture of a copolymer defined according to Claim 1 and one or more polylactic polymer(s).

3. Nanoparticles according to Claim 1 or 2, **characterized in that** they comprise a pharmaceutical active ingredient.

4. Nanopareicles according to any one of Claims 1 to 3, **characterized in that** the copolymer is a polylactic/polyethylene glycol: PLA²⁹⁰⁰-PEG²¹⁰⁰ copolymer.

5. Nanoparticles according to any one of Claims 1 to 3, **characterized in that** the copolymer is a polylactic/polyethylene glycol: PLA⁹⁶⁰⁰-PEG²¹⁰⁰ copolymer.

6. Nanoparticles according to any one of Claims 1 to 5, **characterized in that** they have an average size of between 50 and 500 nm.

7. , Nanoparticles according to Claim 6, **characterized in that** they have an average size of between 50 and 250 nm.

8. Application of the nanoparticles according to any one of Claime 1 to 7, for the preparation of a medicinal product intended for human or animal pharmacy.
